# EUROPÄISCHE PATENTANMELDUNG

(11) **EP 2 042 493 A1**
(43) Veröffentlichungstag der Anmeldung: **01.04.2009**
(21) Anmeldenummer: 07116434.7
(22) Anmeldetag: 14.09.2007
(51) Int. Cl.: C07D 277/18

(54) **Verfahren zum Herstellen von Cyanimino-1,3-thiazolidinen**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zum Herstellen von Cyanimino-1,3-thiazolidinen, welche wichtige Bausteine zur Herstellung von Pflanzenschutzwirkstoffen und Pharmazeutika sind, nach folgendem Schema: 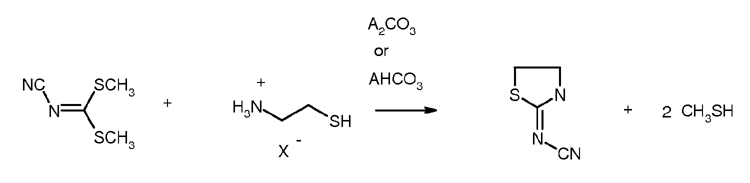 Wobei A ein Alkalimetall ist und X einen Säurerest darstellt.

## Beschreibung

Die vorliegende Erfindung betrifft ein Verfahren zur Herstellung von Cyanimino-1,3-thiazolidinen, welche wichtige Bausteine zur Herstellung von Pflanzenschutzwirkstoffen und Pharmazeutika sind.

Es ist bekannt, dass man Cyanimino-1,3-thiazolidin erhält, wenn man Dimethyl-N-cyanocarbonimidodithiocarbonat und Cysteamin in Ethanol unter Rückfluss erhitzt (vgl. Archiv der Pharmazie 305, 731 (1972) sowie DE 2205745. 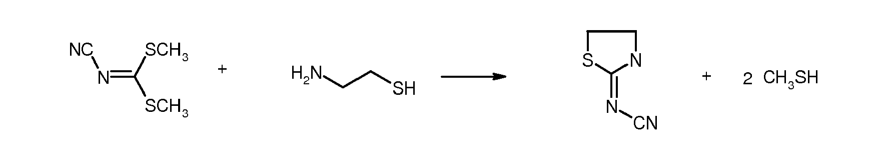 Die dort beschriebenen Ausbeuten sind für eine industrielle Herstellung des Produktes zu niedrig. EP 1 460 068 A1 beschreibt die Umsetzung von Dimethyl-N-cyanocarbonimidodithiocarbonat und Cysteamin in Gegenwart von Alkalimetallhydroxiden. Nachteilig bei diesem Verfahren ist die starke Basizität der Alkalimetallhydroxide, die bei ungenauer Dosierung zu Produktverlusten und damit schlechteren Ausbeuten führt.

Ein weiteres Verfahren wird in Chimia (Chimia 2003, 57, No 11, 710 - 714) beschrieben. Hierbei wird die Umsetzung mit Natriumhydrogencarbonat in Ethanol bei 40°C beschrieben.

Im Hinblick auf die vorstehend geschilderten Nachteile und Probleme besteht der Bedarf, ein Verfahren bereitzustellen, das ausgehend von Dimethyl-N-cyanocarbonimidodithio-carbonat das Cyanoimino-1,3-thiazolidin mit hoher Ausbeuten und hoher Selektivität zugänglich macht.

Ferner besteht der Bedarf, ein Verfahren bereitzustellen, bei welchem die Freisetzung von Methylmercaptan verhindert wird, da dieses toxisch und stark geruchsbelästigend ist.

Gelöst wird diese Aufgabe durch das folgende Verfahren. Dimethyl-N-cyanocarbonimidodithiocarbonat und 2-Aminoethanthiol oder ein Salz hiervon (Formel (I)) 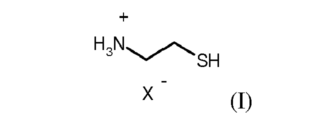 werden in Gegenwart von Wasser und Alkalimetallcarbonaten oder Alkalimetallhydrogencarbonaten zu Cyanimino-1,3-thiazolidin der Formel (II) umsetzt. 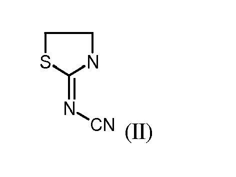 X steht hierbei für einen Säurerest wie beispielsweise Halogen, Acetat, Sulfat oder Hydrogensulfat.

Die Reaktion erfolgt nach dem Schema 1, 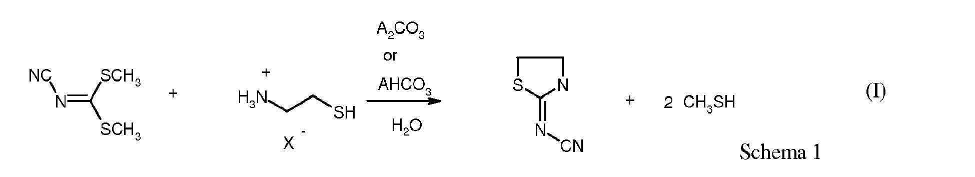 wobei A für ein Alkalimetall steht.

Es ist überraschend, dass in Gegenwart von Alkalimetallcarbonaten bzw. -hydrogencarbonaten und Wasser die Reaktion bei vergleichsweise tiefen Reaktionstemperaturen zwischen 5-15°C abläuft. Das Produkt entsteht hierbei in hoher Ausbeute. Ferner unterbleibt eine gasförmige Freisetzung des giftigen Methylmercaptans, was für eine großtechnische Produktion sicherheitsrelevant ist.

Bevorzugt ist die Durchführung des erfindungsgemäßen Verfahrens unter Verwendung von Verbindungen der Formel (I), in welcher X für einen Säurerest wie beispielsweise Halogen, Acetat, Sulfat oder Hydrogensulfat steht.

### Bevorzugt steht X für Chlorid, Sulfat oder Hydrogensulfat

Für das erfindungsgemäße Verfahren werden Alkalimetallcarbonate oder Alkalimetallhydrogencarbonate eingesetzt. Vorzugsweise werden Natriumhydrogencarbonat, Kaliumhydrogencarbonat, Natriumcarbonat und Kaliumcarbonat eingesetzt. Besonders bevorzugt sind Natriumhydrogencarbonat und Natriumcarbonat.

Die im erfindungsgemäßen Verfahren als Ausgangsstoffe zu verwendenden Cysteaminsalze der Formel (I) sind kommerziell erhältlich und allgemein bekannte Verbindungen der organischen Chemie.

Das erfindungsgemäße Verfahren läuft in Anwesenheit von Wasser ab. Es können auch wasserhaltige Lösungsmittelgemische verwendet werden. Diese können neben Wasser auch andere Lösungsmittel enthalten. Als Beispiele sind zu nennen: Halogenkohlenwasserstoffe, insbesondere Chlorkohlenwasserstoffe, wie Tetrachlorethylen, Tetrachlorethyl, Dichlorpropan, Methylenchlorid, Dichlorbutan, Chloroform, Tetrachlorkohlenstoff, Trichlorethyl, Trichlorethylen, Pentachlorethyl, Difluorbenzol, 1,2-Dichlorethyl, Chlorbenzol, Brombenzol, Dichlorbenzol, Chlortoluol, Trichlorbenzol; Alkohole wie Methylol, Ethylol, Isopropanol, Butanol; Ether, wie Ethylpropylether, Methyl-tert-butylether, *n*-Butylether, Anisol, Phenetol, Cyclohexylmethylether, Dimethylether, Diethylether, Dimethylglycol Diphenylether, Diproplether, Diisopropylether, Di-n-butylether, Diisobutylether, Diisoamylether, Ethylenglycoldimethylether, Isopropylethylether, Methyl-tert-butylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, *N*-Methylmorpholin, Pyridin, alkylierte Pyridine und Tetramethylendiamin; Nitrokohlenwasserstoffe wie Nitromethyl, Nitroethyl, Nitropropan, Nitrobenzol, Chlornitrobenzol, o-Nitrotoluol; Nitrile wie Acetonitril, Methylnitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan; beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N*-Methyl-formamid, *N,N*-Dimethyl-formamid, *N,N*-Dipropyl-formamid, *N,N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin; Ketone wie Aceton, Acetophenon, Methylethylketon, Methylbutylketon.

Außerdem kann das erfindungsgemäße Verfahren in wässrigen Zweiphasensystemen ablaufen. Dabei wird ein weiteres nicht- oder nur sehr begrenzt wassermischbares Lösungsmittel verwendet.

Bevorzugte Lösungsmittel, die zusammen mit Wasser verwendet werden können, sind: Methanol, Ethanol, THF, Butanol.

In einer bevorzugten Ausführungsform besteht das Lösungsmittel zu mindestens 50% aus Wasser.

In einer besonders bevorzugten Ausführungsform besteht das Lösungsmittel zu mindestens 95% aus Wasser.

In einer ganz besonders bevorzugten Ausführungsform wird als Lösungsmittel nur Wasser verwendet.

Das Cysteamin-Hydrochlorid oder das Cysteamin wird in einer Lösung von Alkalimetallcarbonaten oder Alkalimetallhydrogencarbonaten gelöst. Dieser Vorgang kann bei Raumtemperatur erfolgen. Anschließend wird die Lösung auf 5 - 15 °C, bevorzugt 10°C, abgekühlt. Das Dimethyl-N-cyanocarbonimidodithiocarbonat wird zudosiert.

Das molare Verhältnis von Cysteamin-Hydrochlorid zu Dimethyl-N-cyanocarbonimidodithiocarbonat liegt vorzugsweise im Bereich von 1 : 0,7 bis 1 : 1,5. Besonders bevorzugt ist ein Bereich von 1: 0,95 bis 1: 1,05.

Nach Beendigung der Dosierung wird der Reaktionsansatz noch 0,5 - 10 Stunden bei Temperaturen von 10 -15 °C gerührt. Bevorzugt sind 1 - 4 Stunden. Längere Reaktionszeiten sind allerdings unkritisch.

Nach Abschluss der Reaktion kann der pH-Wert der Lösung auf 4 bis 6 mittels einer Säure eingestellt werden. Dieser Schritt ist nicht essenziell für die Durchführung des Verfahrens, kann aber zu höheren Ausbeuten führen. Als Säure kann eine anorganische Säure oder eine organische Säure verwendet werden. Beispielsweise können Salzsäure, Phosphorsäure, Schwefelsäure oder Salpetersäure verwendet werden.

Das erfindungsgemäße Verfahren lässt sich diskontinuierlich oder kontinuierlich durchführen. Ferner lässt sich das Verfahren unter Normaldruck, Unter- oder Überdruck durchführen.

Die Aufarbeitung kann durch Filtration erfolgen. Dadurch wird auch das Methylmercaptan abgetrennt. Eine aufwändige Destillation wie sie im Stand der Technik beschriebenist, ist dadurch nicht notwendig.

Das erfindungsgemäße Verfahren zum Herstellen von Cyanimino-1,3-thiazolidin wird in den nachstehenden Beispielen beschrieben, welche die obige Beschreibung weiter illustrieren. Die Beispiele sind jedoch nicht in einschränkender Weise zu interpretieren.

### Herstellungsbeispiele:

### Beispiel 1

Zu einer Lösung von 26,5 g Natriumcarbonat in 150 ml Wasser werden bei Raumtemperatur 28,8 g Cysteamin-Hydrochlorid zudosiert. Die Reaktionsmischung wird auf 10°C abgekühlt und 37,1 g Dimethyl-N-cyanocarbonimidodithiocarbonat zudosiert. Nach Ende der Dosierung rührt man noch weitere 2 Stunden bei 10°C und erwärmt dann auf 20°C. Bei 20°C werden 37 g 20%ige Salzsäure zugetropft. Die Mischung wird anschließend filtriert und der Feststoff mit 100 ml Wasser gewaschen. Nach Trocknung im Vakuum erhält man 30,3 g Cyanimino-1,3-thiazolidin (entspricht einer Ausbeute 94,7 %).

### Beispiel 2

Zu einer Lösung von 26,5 g Natriumcarbonat in 150 ml Wasser werden bei Raumtemperatur 28,8 g Cysteamin-Hydrochlorid zudosiert. Die Reaktionsmischung wird auf 10°C abgekühlt und 37,1 g Dimethyl-N-cyanocarbonimidodithiocarbonat zudosiert. Nach Ende der Dosierung rührt man noch weitere 2 Stunden bei 10°C und erwärmt dann auf 20°C. Die Mischung wird anschließend filtriert und der Feststoff mit 2 mal mit 100 ml Wasser gewaschen. Nach Trocknung im Vakuum erhält man 29,8 g Cyanimino-1,3-thiazolidin (entspricht einer Ausbeute 93 %).

### Beispiel 3

Zu einer Lösung von 23,8 g Natriumhydrogencarbonat in 200 ml Wasser werden bei Raumtemperatur 28,8 g Cysteamin-Hydrochlorid zudosiert. Die Reaktionsmischung wird auf 10°C abgekühlt und 37,1 g Dimethyl-N-cyanocarbonimidodithiocarbonat zudosiert. Nach Ende der Dosierung rührt man noch weitere 3 Stunden bei 10°C und erwärmt dann auf 20°C. Die Mischung wird anschließend filtriert und der Feststoff mit 100 ml Wasser gewaschen. Nach Trocknung im Vakuum erhält man 29,4 g Cyanimino-1,3-thiazolidin (entspricht einer Ausbeute 92 %).

## Patentansprüche

1. Verfahren zur Herstellung von Cyanimino-1,3-thiazolidin durch Umsetzung von Dimethyl-N-cyanocarbonimidodithiocarbonat und Salzen des Cysteamins in Anwesenheit mindestens eines Alkalimetallcarbonats oder Alkalimetallhydrogencarbonats, wobei das Lösungsmittel zu mindestens 50% aus Wasser besteht.

2. Verfahren gemäß Anspruch 1, wobei als Lösungsmittel nur Wasser verwendet wird.

3. Verfahren gemäß einem der Ansprüche 1 oder 2, wobei die Reaktion in einem Temperaturbereich von 5-15 °C abläuft.

4. Verfahren gemäß einem der Ansprüche 1-3, wobei das Molverhältnis von Dimethyl-N-cyanocarbonimidodithiocarbonat zu Salzen des Cysteamins im Bereich von 1:0,7 bis 1:1,5 liegt.

5. Verfahren gemäß einem der Ansprüche 1-4, wobei die Alkalimetallcarbonate ausgewählt sind aus Natriumcarbonat und Kaliumcarbonat sowie das Alkalimetallhydrogencarbonat ausgewählt ist aus Natriumhydrogencarbonat und Kaliumhydrogencarbonat.
